(19)

**Europäisches Patentamt**
**European Patent Office**
**Office européen des brevets**

(11) **EP 3 020 333 A1**

(12) **EUROPEAN PATENT APPLICATION**

(43) Date of publication:
**18.05.2016 Bulletin 2016/20**

(51) Int Cl.:
**A61B 5/04** *(2006.01)* **A61B 5/046** *(2006.01)*
**A61B 5/00** *(2006.01)*

(21) Application number: **14382456.3**

(22) Date of filing: **17.11.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Fundación Centro Nacional de Investigaciones Cardiovasculares Carlos III (CNIC)**
**28029 Madrid (ES)**
• **Universidad Politecnica de Valencia**
**46022 Valencia (ES)**
• **Fundación para la Investigación Biomédica del Hospital Universitario de la Paz**
**28046 Madrid (ES)**

(72) Inventors:
• **Filgueiras Rama, David**
**28029 Madrid (ES)**
• **López de Sá y Areses, Esteban**
**28046 Madrid (ES)**
• **Millet Roig, José**
**46022 Valencia (ES)**
• **Calvo Saiz, Conrado Javier**
**46022 Valencia (ES)**

(74) Representative: **Hoffmann Eitle**
**Hoffmann Eitle SLU**
**Paseo de la Habana 9-11**
**28036 Madrid (ES)**

(54) **Method of predicting or prognosticating neurological performance in patients who have suffered a cardiac arrest and optionally comatose status due to ventricular fibrillation**

(57) This invention refers to the medical field, in particular to the field of diagnosis and prognosis. Particularly, the invention relates to a method of predicting or prognosticating neurological performance in patients which have suffered a cardiac arrest, and optionally comatose status, due to ventricular fibrillation, wherein the method comprises processing at least one electrocardiogram lead from the patient showing the ventricular fibrillation trace prior to the first direct current shock to generate a Power-Spectral-Density (PSD) spectrum, preferably a power spectral density spectrum using the Welch method, and using as an indicator whether the Dominant Frequency value (DF) of the PSD spectrum is at or above 3.9 Hz or below 3.9 Hz in the PSD spectrum.

EP 3 020 333 A1

**Description**

**Technical field of the invention**

This invention refers to the medical field, in particular to the field of diagnosis and prognosis, more particularly to a method of predicting or prognosticating neurological performance in patients which have suffered a cardiac arrest and optionally comatose status due to ventricular fibrillation.

**Background of the invention**

Both in-hospital and out-of-hospital cardiac arrest due to ventricular fibrillation (VF) are associated with high mortality rates and significant cerebral disability. VF-derived cerebral injury is a very sensitive time-dependent condition with dramatic social and personal consequences. The absence of cerebral blood flow during VF leads to ischemic damage within a few minutes, which increases after reperfusion due to generation of oxygen free radicals and activation of degradation enzymes, together with other mediators.

To date, mild hypothermia is the only therapy that has shown to increase survival rates and functional outcomes in comatose survivors of cardiac arrest due to VF. However, the use of sedative and neuromuscular blocking drugs in cooled patients may mask neurological damage and delay examination. Furthermore, early prognosis within the first 72 h after cardiac arrest remains unreliable, which is especially relevant in those patients undergoing highly specialized intensive care who might not have any hopes for recovery.

Reducing the time to DC (direct current shock) shock after VF onset is vital to restore spontaneous circulation and minimize cerebral injury. However, the exact time in VF is difficult to determine even after witnessed cardiac arrest. Many VF episodes may initiate as ventricular tachycardia and cerebral blood flow might still persist until VF develops. Reliable experimental data from waveform analysis during VF indicate that both spectral dominant frequency (DF) and median frequency decrease after onset of VF. In this sense, retrospective data in patients with out-of-hospital cardiac arrest and VF have shown that a 5.61 Hz DF threshold can serve as a good predictor for 1-year survival after discharge.

However, although a progressive DF decline as myocardial ischemia takes place seems to predict the overall survival rate, this is not necessarily the case as illustrated throughout the present invention. In addition, relying on DF alone may not be accurate in some cases; for instance when the DF peak is close to a certain cut-off value.

Therefore, there is still a need in the state of the art to provide a reliable prediction model capable of prognosticating survival and/or neurological performance in patients who have suffered a cardiac arrest and comatose status due to ventricular fibrillation.

**Brief description of the invention**

Reliable prognostication in comatose survivors after cardiac arrest due to ventricular fibrillation (VF) is very limited, especially in patients undergoing mild hypothermia. Clinical variables alone are inconsistent in predicting cerebral performance and/or survival. Yet, the authors of the present invention have developed and validated a reliable method useful for the early prognosis of favourable neurological performance (FNP) and/or survival (S). The prediction model presented herein combines: i) information of ventricular fibrillation (VF) spectral characteristics registered at the time of the first reported DC shock, and ii) optionally ambulatory patient-specific clinical information. Such spectral and clinical parameters constitute the necessary tools to provide a reliable RS (risk score) of the expected outcome, cerebral performance and survival, at hospital discharge.

**Brief description of the figures**

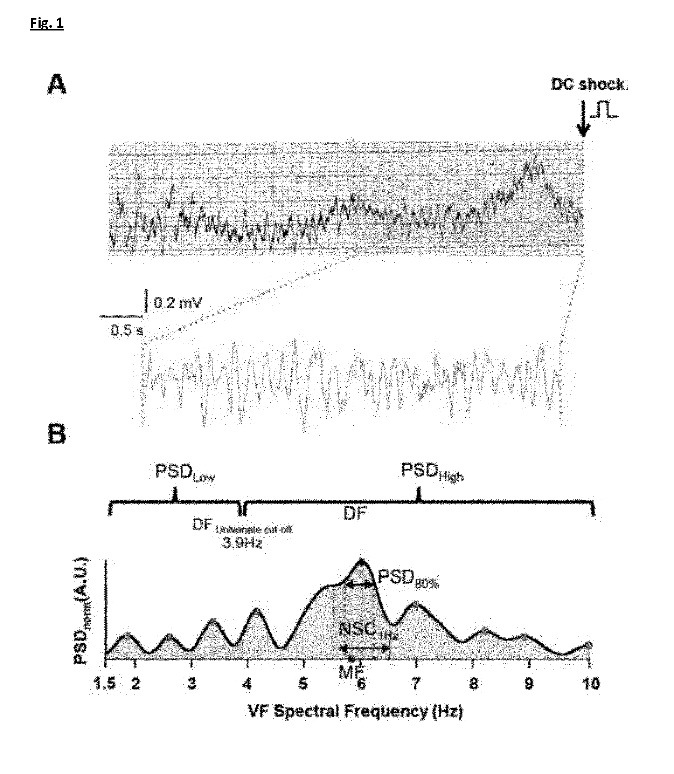

**Figure 1.** Digitization and signal processing of a representative VF trace. A. Upper panel, single lead VF trace from paper ECG prior to the first DC shock. Lower panel, 5-s VF epoch after digitization, segmentation and codification. B. Representative spectra of the VF trace showed in A. DF, MF, 1-Hz DF spectral concentration and PSD 80%, are shown. The univariate cut-off at 3.9 Hz was used to define low and high PSD bands. DF= dominant frequency. MF= median frequency. NSC= normalized spectral concentration. PSD= power spectral density.

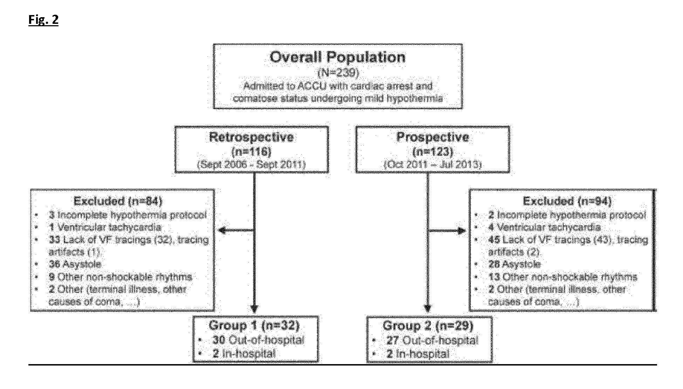

**Figure 2.** Workflow of patients included in group 1 and group 2.

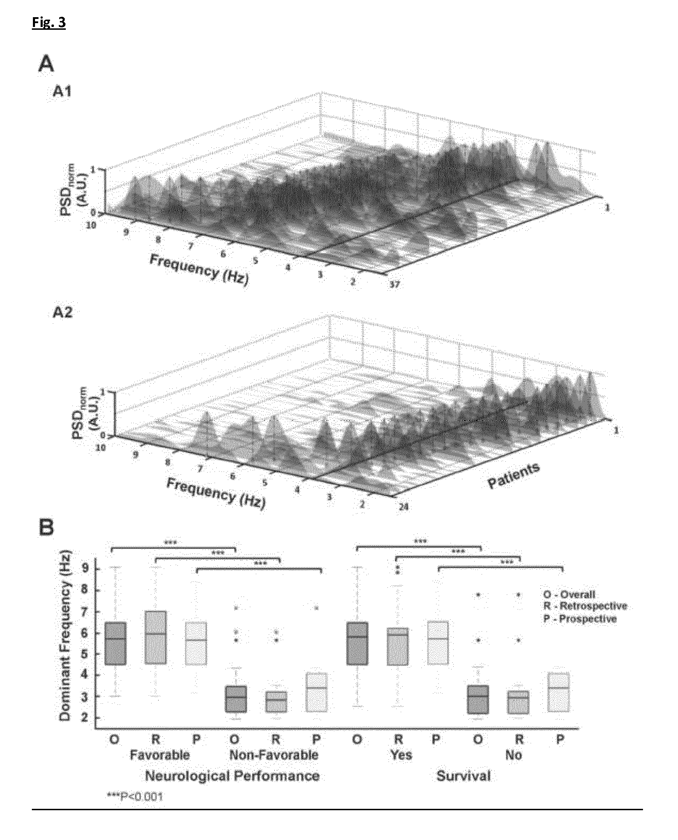

**Figure 3.** A. Power spectral density (PSD) of all patients with in-hospital favorable (A1) and non-favorable (A2) neurological performance. The patients are sorted based on their DF values. DF peaks are pointed out for each individual (black vertical dashed-line). B. Boxplot representation of DF comparing patients from both groups for primary and secondary endpoints. Boxes depict median and interquartile range (25-75%).

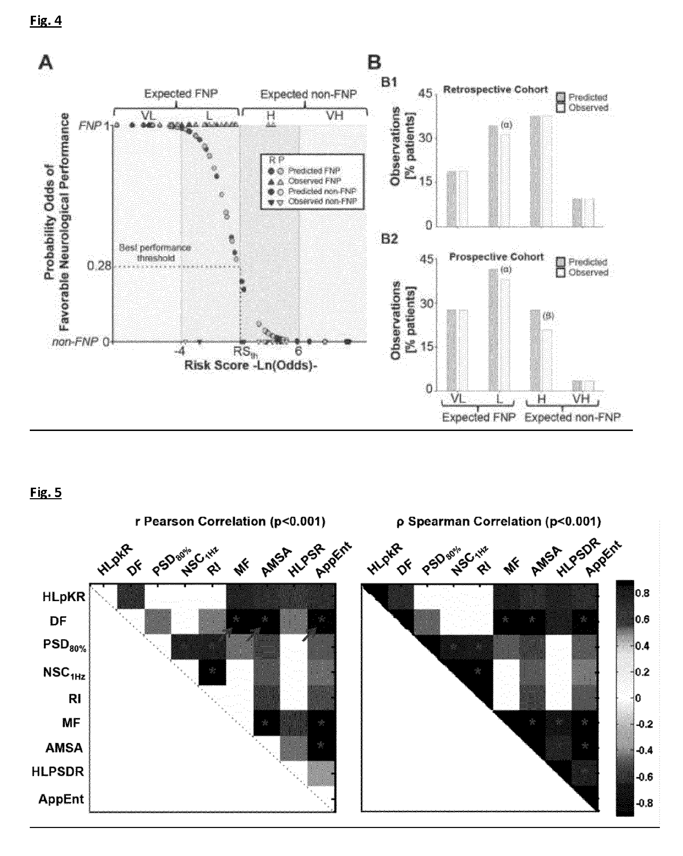

**Figure 4.** Risk score based on the predictive performance of the model. A. Observed (triangles) and predicted (circles) probability of FNP for the entire population. We defined four risk groups of non-FNP performance according to their risk scores as follows: expected FNP; very low (VL) and low risk (L) and expected non-FNP; high (H) and very high risk (VH). B. Percentage of patients (observed, dark gray and predicted, light gray) who belong to each of the risk score groups in both the retrospective (B1) and prospective cohorts (B2). ($\alpha$) and ($\beta$) represent false negative and false positive individuals, respectively. FNP= favorable neurological performance.

**Figure 5.** Correlation analysis of spectral variables. Both linear (R Pearson) and monotonic (Rho Spearman) correlation coefficients were calculated among spectral and temporal ventricular fibrillation variables significantly associated to favorable neurological performance. Arrows highlight strong linear and monotonic correlation between DF and MF, AMSA and AppEnt in the retrospective cohort. AMSA= amplitude spectrum area. AppEnt= Approximate Entropy Regularity Index. DF= dominant frequency. HLpKR= High-to-Low peak ratio. HLPSDR= High-to-Low PSD ratio. MF= median frequency. NSC= normalized spectral concentration. PSD= power spectral density. RI= Spectral regularity index.

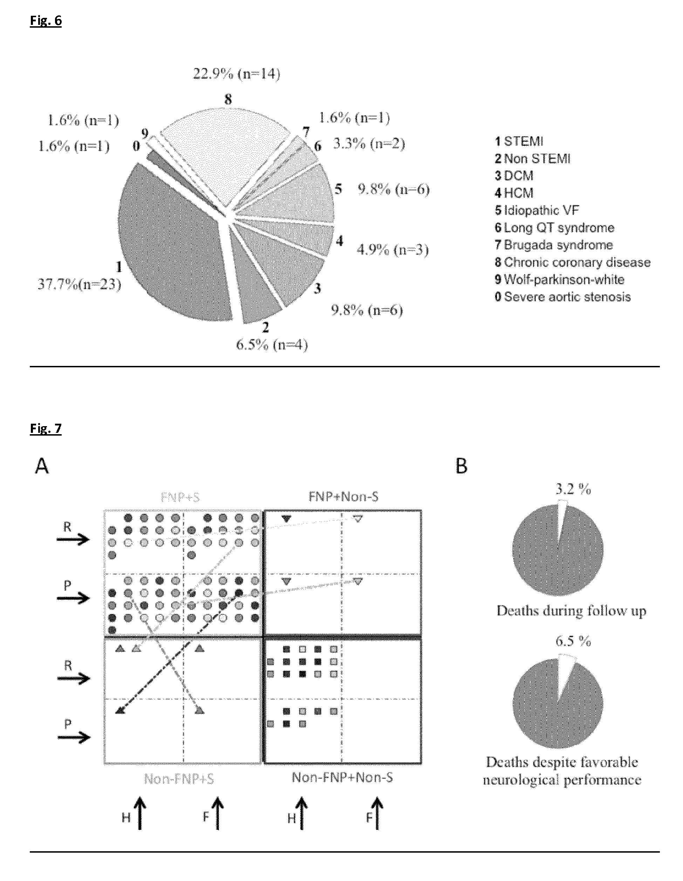

**Figure 6.** Causes of ventricular fibrillation across population (N=61).

**Figure 7.** Individual outcomes and follow-up. **A.** Quadrant-based representation of outcomes and follow-up of each individual patient. Patients are sorted in each quadrant whether they belong to the retrospective (top, R) or prospective (bottom, P) cohorts. Follow-up (F) of patients who survived to hospital discharge (H) is shown on the right for each group. Transitions during follow-up are indicated with a dashed line. Markers represent each quadrant condition (circles; FNP+S, downward triangle, FNP+Non-S, upward triangle; Non-FNP+S and squares; Non-FNP+Non-S). **B.** Pie charts showing deaths at follow-up and deaths despite favorable neurological performance. FNP= Favorable neurological performance. S= Survival.

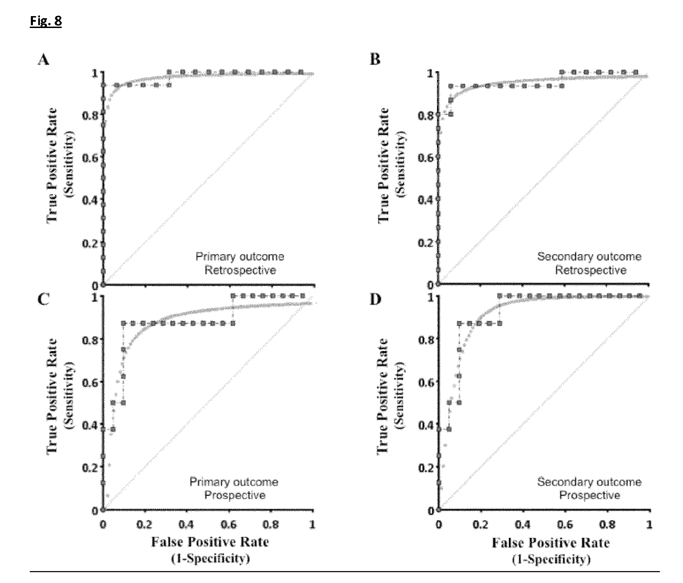

**Figure 8.** ROC curves for the best performance prediction model. **A.** ROC curve for in-hospital favorable neurological performance in the retrospective cohort (C-stat= 0.98). **B.** ROC curve for the secondary endpoint (survival) in the retrospective cohort (C-stat= 0.95). **C.** ROC curve for the primary outcome in the prospective cohort. (C-stat= 0.88). **D.** ROC curved for survival to hospital discharge in the prospective cohort (C-stat= 0.92).

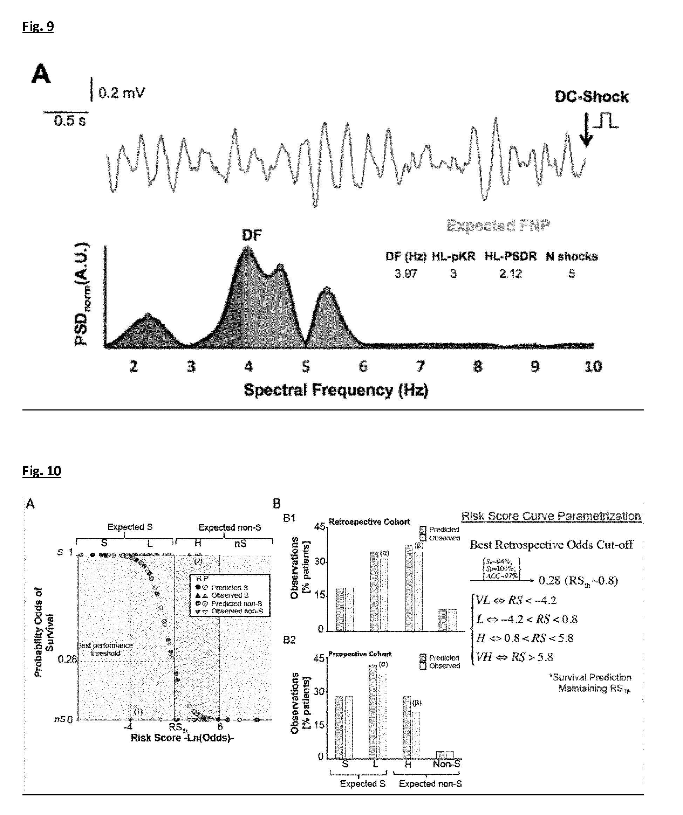

**Figure 9.** Representative VF recordings with a DF peak close to the cut-off value (3.9 Hz). Upper panel, digitized trace before the first DC shock. Lower panel, representative spectrum of the VF signal. Despite a DF peak at 3.97 Hz, both HL-PSDR (2.12) and HLpKR (3) aided to correctly classified such a case within low risk of non-favorable neurological performance: Risk score Log (odds); Log (-1.79). DF= dominant frequency, VF= ventricular fibrillation.

**Figure 10.** Risk score based on the predictive performance of the model tested for Survival prediction at hospital discharge based on the spectral parameters (DF and both HL-PSDR and HLpKR) and the number of shocks delivered before ROSC (NShocks).

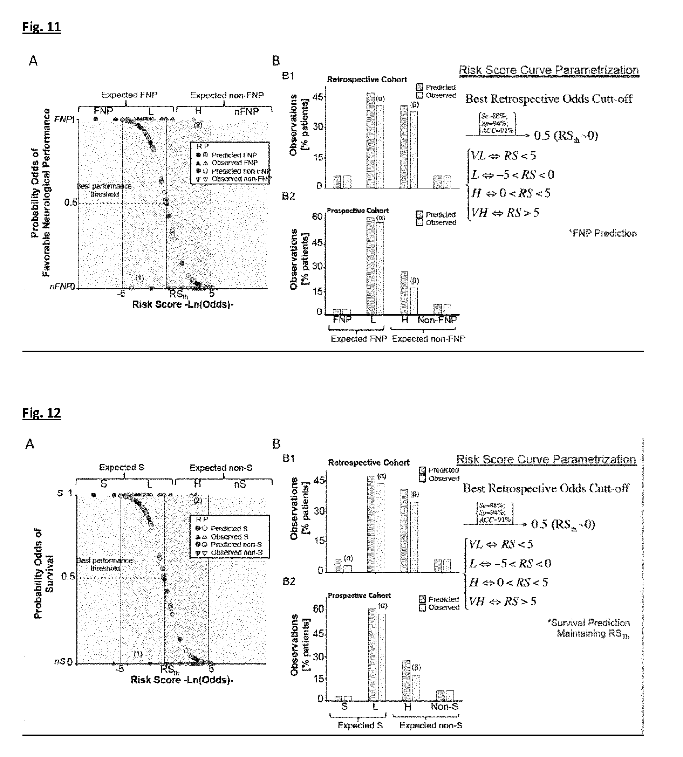

**Figure 11.** Risk score based on the predictive performance of the model for FNP at hospital discharge using only the spectral predictors (DFmax, HLPSDR, HLPkR). A. Observed (triangles) and predicted (circles) probability of favorable neurological outcome for the entire population. Stratification was done according to their risk scores as follows: expected FNP (VL; Very Low risk and L; Low risk) and expected non-FNP (H;

High risk and VH; Very High risk). **B.** Percentage of patients (observed, dark gray and predicted, light gray) who belong to each of the risk score groups in both the retrospective **(B1)** and prospective cohorts **(B2)**. ($\alpha$ or 1) and (ß or 2) represent false negative and positive individuals, respectively. Right, RS cut-off in each group based on 5% fit fall-down.

**Figure 12.** Risk score based on the predictive performance of the model tested for Survival prediction at hospital discharge using only the spectral predictors (DFmax, HLPSDR, HLPkR).

## Detailed description of the invention

Currently, the reliability of early prognosis in comatose survivors after cardiac arrest due to VF is very limited, which severely impairs the ability of physicians to provide accurate information to patients' relatives and to optimize the use of intensive-resource care. Standardization of mild hypothermia delays neurological evaluation and prognostication due to sedation as well as higher rates of misleading biomarker values within the first 24-48 h. Moreover, the large variability of threshold biomarker values used to predict poor outcome and different measurement techniques makes it necessary to exert caution and question the prognostic accuracy provided by biochemical markers.

Clinical variables are also inconsistent in their ability to predict both survival and neurological performance. In this sense, clinical variables such as diabetes, renal failure and myocardial infarction, among others, are not reliable enough to predict outcomes.

Time to cardiopulmonary resuscitation (CPR) after collapse has been shown to correlate with functional outcome. Moreover, when performed properly, CPR seems to improve functional outcome. However, the quality of CPR administered by a bystander might be extremely variable and even if performed by trained personnel it might not add significant improvement in outcome.

Therefore, it is thus clear, that there is still a need to provide a reliable prediction model capable of providing an early prognosis of survival and/or neurological performance in patients which have suffered a cardiac arrest and comatose status due to ventricular fibrillation. In this regard, the authors of the present invention have found a reliable prediction model based on the strong predictive value of DF and certain derived spectral and clinical variables shown herein.

In particular, the authors of the present invention at the time of confronting the above mentioned need, focused primarily on the early prognosis of neurological performance in comatose survivors after cardiac arrest due to VF. In this sense, they noticed that the interval to ALS and total time of ALS reached statistical significance among clinical variables. In addition, all fundamental spectral and time domain VF variables, but VF signal amplitude, were significantly associated to neurological performance ($p<0.05$) (see Table 3). However, surprisingly DF was **strongly** associated with FNP, which was reflected by the best univariate independent predictive accuracy in the retrospective and prospective cohorts (average 0.884).

In fact, a cut-off at 3.9 Hz for DF showed the highest sensitivity (0.88) and specificity (0.94) in predicting the primary endpoint (FNP) in the retrospective cohort. In this sense, the authors of the present invention are the first to demonstrate that the DF value before the first DC shock is a strong independent predictor for FNP when a cut-off value of 3.9 Hz is used. Therefore, the present invention provides a novel practical approach aimed at predicting neurological performance in patients after cardiac arrest due to VF, and optionally comatose status, on admission based on the DF value.

Consequently, a first aspect of the invention refers to a method of predicting or prognosticating neurological performance in patients which have suffered a cardiac arrest, and optionally comatose status, due to ventricular fibrillation, wherein the method comprises processing at least one electrocardiogram lead from the patient showing the ventricular fibrillation trace prior to the first direct current shock to generate a Power-Spectral-Density (PSD) spectrum, preferably a power spectral density spectrum using the Welch method, and using as an indicator whether the Dominant Frequency value (DF) of the PSD spectrum is at or above 3.9 Hz or below 3.9 Hz in the PSD spectrum.

A second aspect of the invention refers to a method of predicting or prognosticating neurological performance in patients which have suffered a cardiac arrest, and optionally comatose status, due to ventricular fibrillation, wherein the method comprises:

> a. processing at least one electrocardiogram lead from the patient showing the ventricular fibrillation trace prior to the first direct current shock, to generate a Power-Spectral-Density (PSD) spectrum, preferably a power spectral density spectrum using the Welch method; and
> b. selecting the highest value from the power frequency spectrum as the Dominant Frequency value (DF);

wherein the result is indicative of a favourable neurological performance if the patient has a Dominant Frequency value (DF) of 3.9 Hz or greater in the PSD spectrum and the result is indicative of a non-favourable neurological performance if the patient has a Dominant Frequency value (DF) of less than 3.9 Hz in the PSD spectrum;
wherein favourable neurological performance is understood as cerebral performance categories (CPC) 1 and 2 (good and moderate disability , respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury; and
wherein the DF value is defined as:

$$DF\,(Hz) = \max\left\{PSD_{ll}(f) \in [1.5-10]Hz\right\}$$

Therefore, based on the previously commented results, it is thus clear that DF alone showed the best univariate independent predictive accuracy amongst spectral variables. However, relying on DF alone may not be accurate in some cases; for instance when the DF peak is close to the cut-off value. In addition, a certain DF value above 3.9 Hz, unfortunately, does not necessarily correlate with survival, which is reflected by the fact that 2 patients during hospitalization and another 2 patients during follow-up died despite FNP in the present study.

Consequently, to increase the predictive accuracy of DF, the authors of the present invention have identified two multivariate models capable of predicting not only neurological performance but also survival in patients after cardiac arrest due to VF, and optionally comatose status, on admission.

For this purpose, the authors of the present invention used the cut-off value of 3.9 Hz to obtain two derived, also significant (p<0.001, see table 3), spectral variables as follows: high to-low power spectral density ratio (HL-PSDR), as the relative power between high (3.9-10 Hz) and low (1.5-3.9 Hz) bands, and high-to-low peak ratio (HL-pKR), as the relative number of spectral peaks above and below 3.9 Hz with power above 40% of the DF (see figure 1).

As illustrated in figures 11 and 12, a risk score based on the predictive performance of the model for FNP and survival prediction at hospital discharge using the spectral predictors: DF, HLPSDR and HLPkR, support the high clinical relevance of this predictive model and risk score. Stratification in these figures was done according to their risk scores as follows: expected FNP (VL; Very Low risk and L; Low risk) and expected non-FNP (H; High risk and VH; Very High risk).

Therefore, a third aspect of the invention refers to a method of predicting or prognosticating neurological performance and/or survival in patients which have suffered a cardiac arrest, and optionally comatose status, due to ventricular fibrillation, wherein the method comprises processing at least one electrocardiogram lead from the patient showing the ventricular fibrillation trace prior to the first direct current shock, to generate a Power-Spectral-Density (PSD) spectrum, preferably a power spectral density spectrum using the Welch method, and using as indicators: i) whether the Dominant Frequency value (DF) of the PSD spectrum is at or above 3.9 Hz or below 3.9 Hz in the PSD spectrum; ii) the high-to-low power spectral density ratio (HL-PSDR), as the relative power between high (3.9-10 Hz) and low (1.5-3.9 Hz) bands; and iii) the high-to-low peak ratio (HL-pKR), as the relative number of spectral peaks above and below 3.9 Hz with a power above 40% of the DF, from the PSD spectrum.

A fourth aspect of the invention refers to a method of predicting or prognosticating neurological performance and/or survival in patients which have suffered a cardiac arrest and comatose status due to ventricular fibrillation, wherein the method comprises:

> a. processing at least one electrocardiogram lead from the patient showing the ventricular fibrillation trace prior to the first direct current shock, to generate a Power-Spectral-Density (PSD) spectrum, preferably a power spectral density spectrum using the Welch method;
> b. selecting the highest value from the power frequency spectrum as the Dominant Frequency value (DF);
> c. selecting the high-to-low power spectral density ratio (HL-PSDR), as the relative power between high (3.9-10 Hz) and low (1.5-3.9 Hz) bands; and
> d. Selecting the high-to-low peak ratio (HL-pKR), as the relative number of spectral peaks above and below 3.9 Hz with a power above 40% of the DF;

wherein the result is obtained by determining a risk score based on the predictive performance of the combination of at least the three variables above mentioned (in steps b) to d)) that classifies the patients in those predicted to have favourable neurological performance or non-favourable neurological performance and/or those predicted to survived or not survived; said risk score can be determined as explained in the materials and methods of example 1 of the present invention;

wherein favourable neurological performance is understood as cerebral performance categories (CPC) 1 and 2 (good and moderate disability, respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury;

wherein survival is understood as the patient being alive at hospital discharge or for at least 6 months after suffering the aforementioned cardiac arrest;

wherein the DF value is defined as:

$$DF\,(Hz) = \max\left\{PSD_{ll}\,(f) \in [1.5-10]Hz\right\}$$

wherein the high-to-low power spectral density ratio (HL-PSDR) is defined as:

$$HLPSDR(A.U.) = \frac{\left.\int_{Th}^{10Hz} PSD_{II}(f)df\right|_{Th=3.9Hz}}{\left.\int_{1.5}^{Th} PSD_{II}(f)df\right|_{Th=3.9Hz}}$$

and wherein the high-to-low peak ratio (HL-pKR) is defined as:

$$HLPkR(A.U.) = \frac{\left. n\left\{\frac{\partial PSD_{II}(f)}{\partial f} = 0, \frac{\partial^2 PSD_{II}(f)}{\partial f^2} < 0\right\}\right|_{\substack{PSD(f)>40\%PSD(DF_{max}) \\ f\in[Th,10Hz],Th=3.9Hz}}}{\left. n\left\{\frac{\partial PSD_{II}(f)}{\partial f} = 0, \frac{\partial^2 PSD_{II}(f)}{\partial f^2} < 0\right\}\right|_{\substack{PSD(f)>40\%PSD(DF_{max}) \\ f\in[1.5Hz,Th],Th=3.9Hz}}}$$

A fifth aspect of the invention refers to a method of predicting or prognosticating neurological performance and/or survival in patients which have suffered a cardiac arrest and comatose status due to ventricular fibrillation, wherein the method comprises:

a. processing at least one electrocardiogram lead from the patient showing the ventricular fibrillation trace prior to the first direct current shock, to generate a Power-Spectral-Density (PSD) spectrum;
b. selecting the highest value from the power frequency spectrum as the Dominant Frequency value (DF);
c. selecting the high-to-low power spectral density ratio (HL-PSDR), as the relative power between high (3.9-10 Hz) and low (1.5-3.9 Hz) bands; and
d. Selecting the high-to-low peak ratio (HL-pKR), as the relative number of spectral peaks above and below 3.9 Hz with a power above 40% of the DF;

wherein the result is obtained by using the following formula to determine the risk score (RS):

$$RS = -0.819 \cdot (DF) - 0.023 \cdot (HLPSDR) - 1.236 \cdot (HLPkR) + 4.446$$

wherein if the risk score is less than 0 the result is indicative of a favourable neurological performance and/or survival and if the risk score value is greater than 0 the result is indicative of a non-favourable neurological performance and non-survival;
wherein favourable neurological performance is understood as cerebral performance categories (CPC) 1 and 2 (good and moderate disability , respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury;
wherein survival is understood as the patient being alive at hospital discharge or for at least 6 months after suffering the aforementioned cardiac arrest;
wherein the DF value is defined as:

$$DF\,(Hz) = \max\{PSD_{II}(f) \in [1.5-10]Hz\}$$

wherein the high-to-low power spectral density ratio (HL-PSDR) is defined as:

$$HLPSDR(A.U.) = \frac{\left.\int_{Th}^{10Hz} PSD_{II}(f)df\right|_{Th=3.9Hz}}{\left.\int_{1.5}^{Th} PSD_{II}(f)df\right|_{Th=3.9Hz}}$$

and wherein the high-to-low peak ratio (HL-pKR) is defined as:

$$HLPkR(A.U.) = \frac{n\left\{\frac{\partial PSD_{II}(f)}{\partial f} = 0, \frac{\partial^2 PSD_{II}(f)}{\partial f^2} < 0\right\}\Big|_{\substack{PSD(f) > 40\% PSD(DF_{max}) \\ f \in [Th, 10 Hz], Th = 3.9 Hz}}}{n\left\{\frac{\partial PSD_{II}(f)}{\partial f} = 0, \frac{\partial^2 PSD_{II}(f)}{\partial f^2} < 0\right\}\Big|_{\substack{PSD(f) > 40\% PSD(DF_{max}) \\ f \in [1.5 Hz, Th], Th = 3.9 Hz}}}$$

In addition, to further increase the predictive accuracy of DF, the authors of the present invention have identified a further multivariate model. In said model, in addition to spectral parameters: DF, HL-pKR and HL-PSDR, the authors of the present invention further identified the number of shocks delivered before ROSC as an additional useful parameter. Based on this further parameter, the authors obtained the best performance model to predict in-hospital FNP and survival. Multivariate adjusted odds ratios for this specific model are shown in Table 4 below.

For the primary endpoint, this further model achieved sensitivity=0.94 and specificity=1 (c-statistic=0.98). Validation on the prospective cohort also showed high sensitivity (0.88) and specificity (0.91) (c-statistic=0.89). The multivariate model achieved sensitivity=0.94 and specificity=0.94 to predict in-hospital survival in the retrospective cohort (c-statistic=0.95). For the secondary endpoint, predictive performance was also high in the prospective group (sensitivity=0.88, specificity=0.91, cstatistic= 0.92). ROC curves of the multivariate model are shown in Figure 8. In-hospital performance of the model is shown in Table 5.

Performance of this model for both outcomes at follow-up also reached high sensitivity, specificity and c-Statistic values as shown in table 7.

Therefore, a sixth aspect of the invention refers to a method of predicting or prognosticating neurological performance and/or survival in patients which have suffered a cardiac arrest, and optionally comatose status, due to ventricular fibrillation, wherein the method comprises processing at least one electrocardiogram lead from the patient showing the ventricular fibrillation trace prior to the first direct current shock, to generate a Power-Spectral-Density (PSD) spectrum, preferably a power spectral density spectrum using the Welch method, and using as indicators: i) whether the <u>Dominant Frequency value</u> (DF) of the PSD spectrum is at or above 3.9 Hz or below 3.9 Hz in the PSD spectrum; ii) <u>the high-to-low power spectral density ratio</u> (HL-PSDR), as the relative power between high (3.9-10 Hz) and low (1.5-3.9 Hz) bands; iii) <u>the high-to-low peak ratio</u> (HL-pKR), as the relative number of spectral peaks above and below 3.9 Hz with a power above 40% of the DF, from the PSD spectrum; and iv) the number of shocks delivered before return of spontaneous circulation (ROSC).

A seventh aspect of the invention refers to a method of predicting or prognosticating neurological performance and/or survival in patients which have suffered a cardiac arrest, and optionally comatose status, due to ventricular fibrillation, wherein the method comprises:

    a. processing at least one electrocardiogram lead from the patient showing the ventricular fibrillation trace prior to the first direct current shock, to generate a Power-Spectral-Density (PSD) spectrum;
    b. selecting the highest value from the power frequency spectrum as the Dominant Frequency value (DF);
    c. selecting the high-to-low power spectral density ratio (HL-PSDR), as the relative power between high (3.9-10 Hz) and low (1.5-3.9 Hz) bands;
    d. Selecting the high-to-low peak ratio (HL-pKR), as the relative number of spectral peaks above and below 3.9 Hz with a power above 40% of the DF; and
    e. Selecting the number of shocks (NShocks) delivered before return of spontaneous circulation (ROSC);

wherein the result is obtained by determining a risk score based on the predictive performance of the combination of at least the four variables above mentioned that classifies the patients in those predicted to have favourable neurological performance or non-favourable neurological performance and/or those predicted to survived or not survived; said risk score can be determined as explained in example 1 of the present invention;
wherein favourable neurological performance is understood as cerebral performance categories (CPC) 1 and 2 (good and moderate disability , respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury;
wherein survival is understood as the patient being alive at hospital discharge for at least 6 months after suffering the aforementioned cardiac arrest;
wherein the DF value is defined as:

$$DF\,(Hz) = \max\left\{PSD_{II}(f) \in [1.5-10]Hz\right\}$$

wherein the high-to-low power spectral density ratio (HL-PSDR) is defined as:

$$HLPSDR(A.U.) = \frac{\int_{Th}^{10Hz} PSD_{II}(f)df\bigg|_{Th=3.9Hz}}{\int_{1.5}^{Th} PSD_{II}(f)df\bigg|_{Th=3.9Hz}}$$

and wherein the high-to-low peak ratio (HL-pKR) is defined as:

$$HLPkR(A.U.) = \frac{n\left\{\frac{\partial PSD_{II}(f)}{\partial f}=0, \frac{\partial^2 PSD_{II}(f)}{\partial f^2}<0\right\}\bigg|_{\substack{PSD(f)>40\%PSD(DF_{max})\\f\in[Th,10Hz],Th=3.9Hz}}}{n\left\{\frac{\partial PSD_{II}(f)}{\partial f}=0, \frac{\partial^2 PSD_{II}(f)}{\partial f^2}<0\right\}\bigg|_{\substack{PSD(f)>40\%PSD(DF_{max})\\f\in[1.5Hz,Th],Th=3.9Hz}}}$$

An eight aspect of the invention refers to a method of predicting or prognosticating neurological performance and/or survival in patients which have suffered a cardiac arrest, and optionally comatose status, due to ventricular fibrillation, wherein the method comprises:

a. processing at least one electrocardiogram lead from the patient showing the ventricular fibrillation trace prior to the first direct current shock, to generate a Power-Spectral-Density (PSD) spectrum;
b. selecting the highest value from the power frequency spectrum as the Dominant Frequency value (DF);
c. selecting the high-to-low power spectral density ratio (HL-PSDR), as the relative power between high (3.9-10 Hz) and low (1.5-3.9 Hz) bands;
d. Selecting the high-to-low peak ratio (HL-pKR), as the relative number of spectral peaks above and below 3.9 Hz with a power above 40% of the DF; and
e. Selecting the number of shocks delivered before return of spontaneous circulation (ROSC);

wherein the result is obtained by using the following formula to determine the risk score (RS):

$$RS = -0.734 \cdot (DF) - 0.215 \cdot (HLPSDR) - 1.145 \cdot (HLPkR) + 0.445 \cdot (NShocks) + 2.781$$

wherein if the risk score is less than 0.8 the result is indicative of a favourable neurological performance and/or survival and if the risk score value is greater than 0.8 the result is indicative of a non-favourable neurological performance and non-survival;
wherein favourable neurological performance is understood as cerebral performance categories (CPC) 1 and 2 (good and moderate disability , respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury;
wherein survival is understood as the patient being alive at hospital discharge or for at least 6 months after suffering the aforementioned cardiac arrest; wherein the DF value is defined as:

$$DF\,(Hz) = \max\left\{PSD_{II}(f) \in [1.5-10]Hz\right\}$$

wherein the high-to-low power spectral density ratio (HL-PSDR) is defined as:

$$HLPSDR(A.U.) = \frac{\left.\int\limits_{Th}^{10Hz} PSD_{II}(f)df\right|_{Th=3.9Hz}}{\left.\int\limits_{1.5}^{Th} PSD_{II}(f)df\right|_{Th=3.9Hz}}$$

and wherein the high-to-low peak ratio (HL-pKR) is defined as:

$$HLPkR(A.U.) = \frac{n\left\{\left.\frac{\partial PSD_{II}(f)}{\partial f} = 0, \frac{\partial^2 PSD_{II}(f)}{\partial f^2} < 0\right\}\right|_{\substack{PSD(f)>40\%PSD(DF_{max}) \\ f\in[Th,10Hz],Th=3.9Hz}}}{n\left\{\left.\frac{\partial PSD_{II}(f)}{\partial f} = 0, \frac{\partial^2 PSD_{II}(f)}{\partial f^2} < 0\right\}\right|_{\substack{PSD(f)>40\%PSD(DF_{max}) \\ f\in[1.5Hz,Th],Th=3.9Hz}}}$$

The dominant frequency in step b) of any of the methods of the present invention might be determined in respect of a period of at least 3 seconds.

The methods of the present invention might be implemented by a computer. Therefore, a further aspect of the invention refers to a computer implemented method, wherein the method is any of the methods disclose herein or any combination therefore.

It is noted that any computer program capable of implementing any of the methods of the present invention or used to implement any of these methods or any combination therefore, also forms part of the present invention.

It is also noted that any device or apparatus comprising or carrying a computer program capable of or, for implementing any of the methods of the present invention or any combination therefore, is included as forming part of the present specification.

Finally, the methods of the present invention may be applied with individuals of either sex, i.e. men or women, and at any age. The profile determined by the present invention is predictive and prognostic.

The following examples serve to illustrate the present invention; these examples are in no way intended to limit the scope of the invention.

**Examples**

**Example 1. Materials and Methods.**

**1.1.Study design.**

The study was performed in a referral center for out-of-hospital cardiac arrest (Hospital Universitario La Paz, Madrid, Spain), in which mild therapeutic hypothermia is routinely used in comatose survivors after the event. The study included consecutive patients who underwent mild hypothermia after cardiac arrest due to VF, eventually with ROSC, and comatose status (Glasgow Coma Scale ≤8) on admission. Patients with witnessed or unwitnessed documented VF were eligible for the study, as long as VF traces before the first DC shock had enough quality and duration (≥3 s) for digitization and analysis of spectral parameters, respectively. We excluded patients with early mortality or hemodynamic instability leading to incomplete 24-h of mild hypothermia, and absence of subsequent withdrawal of sedation to assess cerebral performance. Other exclusion criteria were age <18 years, Glasgow Coma Scale score after ROSC >8, non-shockable or shockable rhythms other than VF, a terminal illness or cognitive deterioration present before the cardiac arrest, and possible causes of coma other than cardiac arrest. The study was divided in two groups, as follows: Group 1 with eligible patients from September 2006 to September 2011 and retrospective data analysis, and Group 2 with eligible patients from October 2011 to July 2013, in whom we prospectively studied the utility of the predictive algorithm developed in Group 1. All data were collected from a prospective registry. The institutional ethics review committee approved prospective analysis of the patients, in accordance with European guidelines for good clinical practice.

**1.2.Hypothermia protocol.**

Patients admitted to the acute cardiac care unit underwent routine neurological evaluation before sedation, drug-induced paralysis and initiation of hypothermia protocol as described elsewhere. Briefly, cooling with intravenous cold saline (<8°C) was initiated on admission. This was followed by direct cooling of the blood using the Icy catheter (ZOLL Medical Corporation, Chelmsford, MA) positioned at the level of the inferior vena cava through the femoral vein. Cooling was set at a maximum rate with a target temperature either at 32, 33 or 34°C, which was maintained during 24 hours. Re-warming was controlled at a set rate of 0.1 to 0.3°C per hour to reach 37°C in 12 to 24 hours. Mechanical ventilation was adjusted to ensure normoxemia and normocapnia. Mean blood pressure was maintained between 85 and 100 mm Hg. Blood glucose level was ensured <180 mg/dL. Limitation of active ALS was considered in patients who remained deeply comatose after 5 days of evolution, as long as it was possible to reach an agreement with their representatives.

### 1.3. Spectral Analysis.

For each patient, we analyzed VF epochs prior to the first DC shock. Digitization was performed using a supervised semi-automatic approach based on region of interest selection, histogram thresholding and intensity transformations. Up to 5-s long segments were extracted after segmentation and signal codification from artifact-free VF tracings. Signals were band-pass filtered between 1.5 and 40 Hz. Quality of extraction was visually inspected by two independent investigators. Averaged power spectral density was obtained at each frequency using the non-parametric Welch method for using fast Fourier transform and normalized to the peak power in the 1.5-10 Hz band for each patient. Both time and frequency domain variables were quantified across patients. Those included VF amplitude over time, amplitude spectral area (AMSA), DF, median frequency, approximate entropy regularity index, spectral regularity index, 1-Hz DF spectral concentration and normalized 80% power spectral density (see Figure 1). Investigators blinded to clinical outcome performed all data analysis, extraction and quantification using custom-made scripts of MATLAB software (V. 2010b, The Mathworks Inc, Natick, MA).

### 1.4. Outcome.

The primary outcome was a favorable neurological performance (FNP) during hospitalization. All patients were classified using the Pittsburgh outcome categorization of brain injury as follows: cerebral performance categories (CPC) 1 and 2 (good and moderate disability, respectively) were considered as FNP, and CPC 3, 4 and 5 (severe disability, vegetative state and brain death, respectively) were considered as a non-FNP. Neurological outcome was established after in-hospital stabilization or before hospital discharge. In patients from group 1, retrospective data were obtained from clinical records during hospitalization.

The secondary outcome measure was survival to hospital discharge and at least 6 months after hospital discharge.

### 1.5. Follow-up.

Neurological outcome was prospectively assessed in all survivors after October 2011, either from group 1 or group 2. Specifically, patients from group 2 were evaluated between 3 and 6 months after hospitalization. Neurological outcome was also determined in both groups using the mini-mental state examination as follows: any score ≥24 points (out of 30) indicated a good cognition. Scores <24 indicated cognitive impairment.14 Only patients with both CPC ≤2 and mini-mental state examination score ≥24 were considered to have FNP at follow-up.

Survival after hospitalization was assessed in group 1 after October 2011. In patients from group 2, survival was assessed at 6 months after hospital discharge.

### 1.6. Statistical Analysis.

All values are presented as median±SEM (25th,75th percentiles) except where noted. The retrospective cohort was used to develop a model for predicting the primary outcome. Each of the clinical, spectral and time domain VF variables underwent univariate analysis to evaluate its association with in-hospital FNP. Normal distribution of variables was assessed with Shapiro-Wilk test. Statistical significance was assessed by the T-test or the Mann-Whitney-Wilcoxon test, as appropriate. If necessary, we used Bonferroni correction for multiple comparisons. Categorical variables and percentile comparisons were compared using a Chi-squared test or the Fisher Exact test, as appropriate. $P < 0.05$ was considered statistically significant. Non-correlated variables, amongst statistically significant ones, and clinical relevant variables were regressed out against the primary outcome by using a stepwise backward multivariate logistic regression approach.

We aimed at predicting in-hospital FNP with the highest sensitivity and specificity achievable using the minimum number of variables. We validated the predictive accuracy of the model in the prospective cohort and tested the model during follow-up. We also studied in both groups the accuracy of the model in predicting survival. Patients from both groups were categorized according to their risk scores obtained in the multivariate analysis. Goodness of fit was assessed through Pearson residuals and Chi-squared deviance. To correct for bias, we obtained bootstrapped standard errors for weights. To guarantee robustness we used the Jackknife fitted regression weights to confirm the minimum mean squared error. All analyses were done using SSPS v21 and custom Matlab scripts for mathematical assistance.

**1.7.Supplemental methods**

**1.7.1. Analysis of ventricular fibrillation traces.**

All data analyses, extraction and quantifications were done using custom scripts written in MATLAB software (version 7.11.0 R2010b, The MathWorks Inc, Natick, Massachusetts, USA). The scripts were developed using the retrospective cohort. Blinded investigators to clinical outcomes performed waveform and spectral analysis of ventricular fibrillation (VF) traces.

**- Image digitization, signal extraction and interpretation.**

Standard ECG tape recordings were used to assess waveform parameters of VF prior to the first documented DC shock in all patients. Lead II traces were extracted unless artifacts were present. A semi-automatic approach was used for digitization using a Matlab-based custom tool. VF recordings were scanned to a digital image (1200 dpi) and stored in a codified digital format. To identify VF traces on the standard ECG-paper, image-processing techniques were applied involving binary thresholding, pixel-to-point conversion and transform techniques. When necessary, images were pre-processed to avoid artifacts (contrast, median filtering and interpolation) in the digitization process. Pixel-to-point conversion was applied using the lower waveform envelope. The graphical grid was used as reference for interpretation. Before signal processing, an independent investigator reviewed all digitized traces to ensure accurate extraction of VF deflections.

**- Signal processing.**

Digitized signals were post-processed and further analyzed in Matlab. Up to 5-s artifact-free segments prior to the external DC shock were extracted in each patient. Signals with less duration were zero-padded up to 5 s. Only segments of at least 3 s were eligible for the study (Mean$\pm$Std duration 3.91$\pm$0.98 s, N=61). All signals were detrended by mean removal. A 4th order polynomial approximation was applied to remove the baseline trend when appropriate. Then, VF tracings were bandpass filtered between 1.5 and 40 Hz using 10th order Butterworth digital filters.
Averaged power spectral density (PSD) was obtained at each frequency. We used a nonparametric Welch method for robust Fast Fourier Transform (FFT) estimation with 0.2 Hz spectral resolution in the 1.5 to 10 Hz band of interest, where at least 90% of the spectral power was concentrated. Spectral information in this band was normalized to the total power for each patient before attempting to extract any spectral variable.

**- Extracting temporal and spectral variables.**

Both time and frequency domain pre-selected variables were extracted and quantified across patients. Fundamental spectral variables for VF were measured and calculated as described elsewhere. Dominant frequency (DF) was defined as the frequency with the highest power in the 1.5 to 10 Hz band of interest. Median frequency (MF) describes the power distribution in the frequency spectrum and it was calculated as the frequency cut-off where 50% of the power in the spectrum was below (and above) in the 1.5-10Hz band. Normalized spectral concentration at DF, was obtained both at 80% of the maximum PSD (PSD80%) and through all power density contained in the band DF$\pm$1Hz (NSC).[7] Spectral regularity index (RI) was defined as the ratio between the power at DF and the power in the 1.5 to 10 Hz band.[8] The Amplitude Spectral Area (AMSA) was calculated as the summed product of contributing frequencies weighted by the absolute value of power at that frequency from the PSD Welch spectrum (1.5 to 10 Hz band).
After univariate binary logistic regression analysis on the retrospective cohort, DF offered the best independent prediction accuracy (proportion of true results, both true positives and true negatives, in the population). A cut-off at 3.9 Hz showed the highest sensitivity (0.88) and specificity (0.94) for predicting the primary endpoint in the retrospective cohort. Therefore, we used the 3.9 Hz cut-off to obtain two additional derived ratios of spectral variables as follows: High-to-Low Power Spectral Density Ratio (HL-PSDR) as the relative power between high (3.9 to 10 Hz) and low (1.5 to 3.9 Hz) bands, and High-to-Low Peak Ratio (HL-pKR) as the relative the number of spectral peaks above and below the 3.9 Hz threshold with power above 40% the frequency with the highest power (DF).
We also considered time-domain waveform variables for the study. We included the mean amplitude over time (A) and the Approximate Entropy Regularity Index (AppEnt), which measures the complexity of a time sequence as the natural logarithm of the relative prevalence of repetitive patterns found within the sequence. For multivariate analysis, we selected non-correlated statistically significant variables among all extracted VF fundamental variables. Spearman and Pearson correlations were done to determine monotonic and linear relationships in the retrospective cohort (p<0.001). Highly correlated (Rho and R>0.9) variables were considered for elimination (see Figure 5), leaving those with the highest univariate prediction accuracy for the primary outcome.

**1.7.2. Statistical analysis and prediction model.**

All values are presented as median±SEM (25th,75th percentiles) except where noted. Group 1 (retrospective cohort) was used to develop an algorithm to predict the primary outcome. Each of the clinical, spectral and time-domain VF variables underwent univariate analysis to evaluate its association with in-hospital FNP. Normal distribution of variables was assessed with Shapiro- Wilk test. Statistical significance was assessed by the parametric T-test or non-parametric Mann- Whitney-Wilcoxon test, as appropriate. If necessary, we used Bonferroni correction for multiple comparisons. Categorical variables and percentile comparisons were performed using a Chisquared test or Fisher Exact test, as appropriate. P<0.05 was considered statistically significant. Non-correlated variables, amongst statistically significant ones, and clinically relevant variables were regressed out against the primary outcome by using a stepwise backward multivariate logistic regression approach. All variables were standardized before entering into the multivariate analysis. We considered clinically relevant variables to avoid bias, even though some ended up non-significant or not associated to the primary outcome. Interestingly, the multivariate analysis resulted in the same prediction model either including all relevant clinical variables or not. Based on the prior correlation results and clinical relevance, all variables considered in the multivariate analyses were the following:

- **Clinical variables:** Atrial fibrillation, heart failure, number of shocks delivered before return of spontaneous circulation (ROSC), time to advance life support (ALS), time performing ALS, age, previous myocardial infarction, previous stroke, chronic renal failure.

- **Fundamental and derived VF variables** with the highest univariate prediction accuracy among highly correlated variables: DF, HL-pKR, HL-PSDR, NSC, PSD80%.

The multivariate logistic regression was performed using a backward iterative approach to eliminate variables and search for the best performance model, which was determined by the ROC curves obtained for each classification (maximum squared root product of sensitivity and specificity and maximum C-stat). We aimed at predicting the primary outcome with the highest sensitivity and specificity achievable using the minimum number of variables that guarantied the best performance and predictive accuracy (ACC, percentage of correctly classified observations). Such a predictive model was validated on the prospective cohort. The model was also tested on both cohorts to predict outcomes at follow-up.

To correct for bias, confidence intervals (95%) were found using bias corrected and accelerated bootstrap. Robustness of the correlation between the outcome and expected outcome was challenged using the Jackknife approach to ensure stability beyond individual observations showing that the classification was significantly different from a random classification (p<0.001).

Finally, patients from both groups were categorized according to their risk scores obtained in the multivariate analysis. Using the best probability threshold obtained by the prediction model on the retrospective cohort we defined four risk subsets of the population for non-FNP as follows: very low and low risk of non-FNP (expected FNP), high and very high risk of non-FNP (expected non-FNP). All analyses were done using SSPS v21 and custom Matlab scripts for mathematical assistance.

**Example 2. Results.**

**2.1. Study workflow**

The workflow of the study is depicted in Figure 2. A total of 239 patients undergoing mild hypothermia (n=116, retrospective cohort and n=123, prospective cohort) were assessed for eligibility during the study period. Sixty-one patients (n=31, group 1 and n=29, group 2) fulfilled the inclusion criteria. The vast majority of patients were included after out-of hospital cardiac arrest (n=57). However, two patients in each group were included after in-hospital cardiac arrest due to VF, since comatose status was present after DC shock and ROSC. Baseline clinical characteristics and background treatment of both groups are shown in Table 1 below.

**Table 1.** Baseline Clinical Characteristics

| Clinical Characteristics | Retrospective (n=32) | Prospective (n=29) | Overall (n=61) | P |
|---|---|---|---|---|
| Age(years) | 63.5±4.5 | 55±5.7 | 55±3.72 | 0.0409 |
| Male,n(%) | 31 (96.9) | 23 (79.4) | 54 (88.5) | 0.031 |
| Family History of SCD, n(%) | 1 (3.57) | 8 (40.0) | 9 (15.8) | 0.009 |
| Hypertension, n(%) | 13 (40.6) | 15 (51.7) | 28 (45.9) | 0.385 |
| Dyslipedemia, n(%) | 9 (28.1) | 13 (44.8) | 22 (36.1) | 0.174 |
| Diabetes, n(%) | 7 (21.9) | 3 (10.3) | 10 (16.4) | 0.224 |
| Smoking Habit, n(%) | 12 (37.5) | 13 (44.8) | 22 (36.1) | 0.561 |
| Atrial Fibrillation, n(%) | 5 (15.6) | 6 (20.7) | 11 (18) | 0.849 |
| Heart Failure, n(%) | 10 (31.3) | 6 (20.7) | 12 (26.2) | 0.234 |
| Previous Myocardial Infarction, n(%) | 6 (18.8) | 8 (27.6) | 14 (22.9) | 0.654 |
| Previous Revascularization, n(%) | 4 (12.5) | 4 (13.8) | 6 (13.1) | 0.402 |
| Previous Stroke, n(%) | 1 (3.1) | 1 (3.4) | 2 (3.3) | 0.943 |
| Chronic Renal Failure, n(%) | 2 (6.3) | 0 (0) | 2 (3.3) | 0.271 |
| DCM, n(%) | 2 (6.3) | 0 (0) | 2 (3.3) | 0.17 |
| COPD, n(%) | 3 (9.4) | 5 (17.2) | 8 (13.1) | 0.363 |
| HCM, n(%) | 0 (0) | 3 (10.3) | 3 (4.9) | 0.101 |
| Severe Valvulopathy, n(%) | 2 (6.3) | 1 (3.4) | 3 (4.9) | 0.613 |
| Preexcitation, n(%) | 0 (0) | 1 (3.4) | 1 (1.6) | 0.289 |
| Time to ALS, (min) | 8±24 | 8±2.0 | 8±1.5 | 0.887 |
| Time performing ALS, (min) | 15±5.9 | 10±5.3 | 15±3.9 | 0.282 |
| Number of shocks delivered before ROSC | 3.5±1.6 | 3±0.87 | 3±0.9 | 0.185 |
| **Background Treatment** | | | | |
| Aspirin, n(%) | 4 (12.5) | 5 (17.2) | 9 (14.7) | 0.602 |
| Thienopyridines, n(%) | 1 (3.1) | 0 (0) | 1 (1.6) | 0.524 |
| Betablockers, n(%) | 7 (21.9) | 9 (31) | 16 (26.2) | 0.416 |
| Amiodarone. n(%) | 2 (6.3) | 0 (0) | 2 (3.3) | 0.271 |
| ACE Inhibitors, n(%) | 6 (18.8) | 7 (24.1) | 13 (21.3) | 0.557 |
| ARBs, n(%) | 1 (3.1) | 3 (10.3) | 4 (6.6) | 0.239 |
| Statins, n(%) | 8 (25) | 10 (34.5) | 18 (29.5) | 0.417 |
| Calcium Antagonists, n(%) | 1 (3.1) | 2 (6.9) | 3 (4.9) | 0.496 |
| Diuretics, n(%) | 8 (25) | 6 (20 7) | 14 (22.9) | 0.689 |
| Aldosterone Inhibitors, n(%) | 1 (3.1) | 2 (6.9) | 3 (4.9) | 0.476 |
| Anticoagulants, n(%) | 5 (15.6) | 6 (20.7) | 12 (19.7) | 0.849 |

ACE= angiotensin-converting enzyme ALS= advanced life support, ARBs= Angiotensin-receptor blockers.
COPD= chronic obstructive pulmonary disease DCM= Dilated cardiomyopathy HCM= Hypertrophic cardiomyopathy, ROSC= Return of spontaneous circulation

Female sex, family history of sudden cardiac death and younger age were more frequent in group 2. Overall, the main cause of VF was coronary heart disease, either acute coronary syndromes (n=27, 45%) or chronic coronary disease (n=14, 23%), followed by idiopathic VF (n=6, 10%) and dilated cardiomyopathy (n=6, 10%) (see Figure 6).

**2.2. Outcomes.**

Primary and secondary outcomes are shown in Table 2 below.

**Table 2.** Primary and Secondary Outcomes

| Outcome | Retrospective (n=32) | Prospective (n=29) | Overall (n=61) | P(X2 Test) |
|---|---|---|---|---|
| **Favorable Heurological Performance** | | | | |
| In-Hospital | 13(50.00%) | 21(72.41%) | 37(60.66%) | 0.074 |
| Follow-Up* | 15(46.88%) | 19(65.52%) | 34(55.74%) | |
| **Survival** | | | | |
| Hospital discharge | 17(53.12%) | 21(72.41%) | 38(62.30%) | 0.121 |
| Follow-Up* | 16(50.00%) | 20(68.97%) | 36(59.02%) | |

*Median±SEM (25th, 75th percenttiles). Retrospective: 48.5±10.5 (27.0,68.7) months Prospective 5±1.8 (3.5,7.5) months.

Sixteen patients in group 1 (50.0%) and 21 patients in group 2 (72.4%) achieved FNP during hospitalization. Seventeen patients in group 1 (53.1%) and 21 patients in group 2 (72.4%) survived to hospital discharge. After a median follow-up of 48.5±10.5 months (7.0, 68.7) in group 1, 16 patients (50.0%) were still alive, albeit 15 (48.8%) showed FNP. In group 2, 20 patients (68.8%) were alive at 6 months after hospital discharge and 19 (65.5%) showed FNP after 5±1.8 months (3.5, 7.5). Hospitalization outcomes were not statistically different between groups (Table 2). No patients were missed during follow-up. Outcomes and follow-up of each individual patient are depicted in Figure 7. Four patients died (6.5%) despite FNP. There was a statistically significant association between FNP and survival (see Table 6 below) in both groups (p<0.001).

*Table 6.*

| | | Retrospective (n=32) | | Prospective (n=29) | | Overall (n=61) | |
|---|---|---|---|---|---|---|---|
| | | **Neurological Performance** | | **Neurological Performance** | | **Neurological Performance** | |
| | | Favorable | Non-Favorable | Favorable | Non-Favorable | Favorable | Non-Favorable |
| **Survival** | **Y** | 15 | 2 | 20 | 1 | 35 | 3 |
| | **N** | 1 | 14 | 1 | 7 | 2 | 21 |
| | | **P(FE)** | <0.001 | **P(FE)** | <0.001 | **P(X2)** | <0.001 |

### 2.3. Prediction model.

In creating the model we only considered the primary endpoint since mortality may occur in patients with FNP due to other causes non-directly related with cardiac arrest injury. The interval to ALS and total time of ALS reached statistical significance among clinical variables. All fundamental spectral and time domain VF variables, but VF signal amplitude, were significantly associated to the primary endpoint (p<0.05) (Table 3). Interestingly, DF was strongly associated with FNP, which was reflected by the best univariate independent predictive accuracy in the retrospective and prospective cohorts (average 0.884) (see Table 3 below).

**Table 3.** Univariate Analysis and Independent Predictive Accuracy

| | Neurological Performance | Univariate Unadjusted Odds Ratio | | |
|---|---|---|---|---|
| Categories | P Value | OR (CI 95%) | Univariate P | ACC (CI 95%)* |
| **Clinical Variables** | | | | |
| Age | 0.11 | | | |
| Gender | 0.309 | | | |
| Hypertension | 0.281 | | | |
| | 0.694 | | | |
| Diabetes | 0.199 | | | |
| Smoking Habit | 0.465 | | | |

(continued)

| | Neurological Performance | Univariate Unadjusted Odds Ratio | | |
|---|---|---|---|---|
| Categories | P Value | OR (CI 95%) | Univariate P | ACC (CI 95%)* |
| Atrial Fibrillation | 0.07 | 2.158(0.864 - 5.391) | 0.099 | 0.672(0.506-0.838) |
| Heart Failure | 0.063 | 1.940(0.945-3.986) | 0.071 | 0.655(0.486-0.824) |
| Previous Myocardial Infarction | 0.233 | | | |
| | 0.219 | | | |
| Previous Stroke | 0.309 | | | |
| Chronic Renal Failure | 0.516 | | | |
| DCM | 0.144 | | | |
| COPD | 0.544 | | | |
| HCM | 0.565 | | | |
| Severe Valvulopathy | 0.516 | | | |
| Number of Shocks Delivered before ROSC | 0023 | 3.025(0.991 - 9.229) | 0.052 | 0.671(0.503-0.837) |
| Time to ALS | <0.001 | 10.312(2.044 - 52.032) | 0.005 | 0.689(0.524-0.851) |
| Time Performing ALS | 0.007 | 2.770(1.046 - 7.336) | 004 | 0.676(0.509-0.84) |
| **Background Treatment** | | | | |
| Aspirin | 0.285 | | | |
| Thienopyridines | 0.309 | | | |
| Betablockers | 0.669 | | | |
| | 0.144 | | | |
| ACE Inhibitors | 0.365 | | | |
| ARBs | 0.309 | | | |
| Statins | 0.414 | | | |
| Calcium Antagonists | 0,31 | | | |
| Diuretics | 0.314 | | | |
| Anticoagulants | 0.133 | | | |
| | 0.309 | | | |
| **VF Variables** | | | | |
| **Spectral Domain** | | | | |
| **Fundamental Spectral Variables** | | | | |
| | <0.001 | 0.089(0.020 - 0.403) | 0.002 | 0.884(0.77-0.988) |
| Median Frequency | <0.001 | 0.073(0.015 - 0.355) | 0.001 | 0.86(0.741-0.98) |
| Normalized 80% PSD | 0.005 | 3.121(1.270 - 7.669) | 0.013 | 0.579(0.4-0.75) |
| 1Hz DF Spectral Concentration | 0.023 | 2.198(1.027 - 4.704) | 0.042 | 0.634(0.463-0.805) |
| Amplitude Spectrum Area (AMSA) | <0.001 | 0.053(0.008 - 0.362) | 0003 | 085(0723-0977) |
| Spectral Regularity Index | 0.07 | | | |
| **Derived Spectral Variables** | | | | |
| Higli-to-Low Peak Ratio | <0.001 | 0.073(0.014 - 0.372) | 0.002 | 0.817(0.618-0.954) |
| High-to-Low PSD Ratio | <0.001 | 0.034(0.003 - 0.396) | 0.007 | 0.847(0.721-0.969) |
| **Time Domain** | | | | |
| Mean Amplitude | 0.11 | | | |

(continued)

| Categories | Neurological Performance | | Univariate Unadjusted Odds Ratio | | |
| --- | --- | --- | --- | --- | --- |
| | P Value | OR (CI 95%) | Univariate P | ACC (CI 95%)* | |
| index | <0.001 | 0.032(0.003 - 0.355) | 0.005 | 0.796(0.652-0.937) | |

* Univariate predictive accuracy (ACC) averaged for training (Group 1, retrospective) and validation (Group 2, prospective) OR represent univariate odds ratios AMSA= amplitude spectrum area PSD= Power spectral density. ROSC= return of spontaneous circulation. Other abbreviations as in Table 1

A cut-off at 3.9 Hz showed the highest sensitivity (0.88) and specificity (0.94) in predicting the primary endpoint in the retrospective cohort. Therefore, we used such a cut-off value to obtain two derived, also significant (p<0.001. Table 3), spectral variables as follows: highto- low power spectral density ratio (HL-PSDR), as the relative power between high (3.9-10 Hz) and low (1.5-3.9 Hz) bands, and high-to-low peak ratio (HL-pKR), as the relative number of spectral peaks above and below 3.9 Hz with power above 40% of the DF (Figure 1). Graphic representation of individual spectra and DF peaks of the entire population are shown in Figure 3A. The vast majority of patients with FNP during hospitalization showed DF values above 3.9 Hz (Figure 3A1), unlike those individuals with non-FNP, who had DF values below 3.9 Hz (Figure 3A2). Such differences were statistically significant both in the retrospective and prospective cohorts, as well as in the entire population (p<0.001. Figure 3B). Moreover, DF values also showed significant differences between patients who survived and those who did not survive to hospital discharge (p<0.001. Figure 3B).

Multivariate analysis identified DF, HL-pKR, HL-PSDR and optionally the number of shocks delivered before ROSC as the best performance model to predict in-hospital FNP. Multivariate adjusted odds ratios are shown in Table 4 below.

Table 4. Multivariate Best Performance Model

| Variable | Multivariate adjusted Odds Ratio | CI 95% |
| --- | --- | --- |
| Dominant Frequency | 0.252 | 0.227-0.281 |
| High-to-Low Peak Ratio | 0.164 | 0.145-0.185 |
| High-to-Low PSD Ratio | 0.264 | 0.237-0.295 |
| Number of shocks delivered before ROSC | 5.14 | 4.79-5.51 |
| Abbreviations as in Table 3. | | |

For the primary endpoint, the model achieved sensitivity=0.94 and specificity=1 (c-statistic=0.98). Validation on the prospective cohort also showed high sensitivity (0.88) and specificity (0.91) (c-statistic=0.89). The multivariate model achieved sensitivity=0.94 and specificity=0.94 to predict in-hospital survival in the retrospective cohort (c-statistic=0.95). For the secondary endpoint, predictive performance was also high in the prospective group (sensitivity=0.88, specificity=0.91, cstatistic= 0.92). ROC curves of the multivariate model are shown in Figure 8. In-hospital performance of the model is shown in Table 5 below.

Table 5. In-hospital Performance of the Best Multivariate Predictive Model

| | Sensitivity | Specificity | C-Stat | ACC |
| --- | --- | --- | --- | --- |
| **Favorable Neurological Performance** | | | | |
| Retrospective group (Training) | 0.94 | 1 | 0.98 | 0.97 |
| Prospective group (Validation) | 0.88 | 0.91 | 0.89 | 0.90 |
| **Survival Outcome (Test)** | | | | |
| Retrospective group | 0.94 | 0.94 | 0.95 | 0.938 |
| Prospective group | 0.88 | 0.91 | 0.92 | 0.897 |

Performance of the model for both outcomes at follow-up also reached high sensitivity, specificity and c-Statistic values. (see Table 7 below)

*Table 7*

|  | Se | Sp | C-Stat | ACC |
|---|---|---|---|---|
| **Favorable Neurological Performance** | | | | |
| Retrospective group | 1 | 1 | 1 | 1 |
| Prospective group | 0.88 | 0.91 | 0.90 | 0.90 |
| **Survival Outcome (Test)** | | | | |
| Retrospective group | 0.88 | 0.94 | 0.90 | 0.90 |
| Prospective group | 0.78 | 0.90 | 0.89 | 0.89 |

**2.4. Risk score based on the predictive performance of the model.**

We used the best performance threshold obtained by the prediction model to define fourrisk subsets of the population for non-FNP, as follows: very low and low risk of non-FNP (expected FNP), high and very high risk of non-FNP (expected non-FNP). Interquartile ranges of individual variables within each of the risk score groups are shown in Table 8 below.

EP 3 020 333 A1

**_Table 8_**

| Risk Score Group | Best Performance Predictors | | | | | | | |
|---|---|---|---|---|---|---|---|---|
| | DF(Hz) | | HL-pKR (A.U.) | | HL-PSDR(A.U.) | | Number of shocks before ROSC | |
| | Median±SEM | Percentiles [p25,p75] | Median±SEM | Percentiles [p25,p75] | Median±SEM | Percentiles [p25,p75] | Median±SEM | Percentiles [p25,p75] |
| VL | 6.35±0.75 | [5.42,7.35] | 2±0.46 | [1,2] | 12.3±3.79 | [6.65,16,09] | 1.5±0.78 | [1,3] |
| L | 5.65±0.53 | [4.49,6.17] | 1±0.45 | [1,2] | 3.47±1.04 | [1.84,5.84] | 3±0.67 | [2,4] |
| H | 3.12±0.40 | [2.5,3.48] | -1±0.39 | [-15,-0.5] | 0.35±0.86 | [0.19,1.0] | 5±1.58 | [2.5,5.5] |
| VH | 2.33±0.28 | [2.16,2.64] | -1.5±0.56 | [-2,-1] | 0.34±0.14 | [0.21,0.42] | 13±3.62 | [10.5,16.5] |

DF: Dominant frequency. HL-pKR: High-to-low peak ratio. HL-PSDR: High-to-low power spectral density ratio. Risk score group: L; Low, VL; Very low, H; High and VH; Very high.

Figure 4A shows the observed and predicted probability of in-hospital FNP for the entire population. The risk score correctly classifies more than 93% of observations. Only 1 patient in the retrospective cohort was classified as FNP, although the patient was non-FNP during hospitalization (False negative. Figure 4B1). Three patients in the prospective cohort were identified as false negative and false positives (1 and 2 patients, respectively). The risk score was very reliable in predicting neurological performance in the subgroups of very low and very high risk of non-FNP (Figure 4, B1 and B2). Multivariate adjusted logistic regression weights and statistics for each of the four variables included in the risk score are shown in Table 9 below.

*Table 9*

| Variables | Multivariate Adjusted Non-standardized LR Weight Values | Statistics | | | |
|---|---|---|---|---|---|
| | | Favorable Neurological Performance | | Non-Favorable Neurological Performance | |
| | | Median±SEM | Percentiles [p25,p75] | Median±SEM | Percentiles [p25,p75] |
| Intercept | 2.781 | - | - | - | - |
| Dominant Frequency(Hz) | -0.734 | 5.951±0.475 | [4.51,6.47] | 2.952±0.417 | [2.31,3.48] |
| High-to-Low Peak Ratio | -1.145 | 1±0.381 | [1,2] | -1±0.451 | [-2,-0.5] |
| High-to-Low PSD Ratio | -0.215 | 5.061±2.125 | [2.05,9.93] | 0.335±0.911 | [0.19,1.05] |
| Number of shocks delivered before ROSC | 0.445 | 3±1 | [2,4] | 5±2 | [2.5,7] |

Random representative examples for each one of the risk score groups are shown in Table 10 below.

*Table 10*

| Group Risk Score | | Neurological Performance Observed/Predicted | Predictors | | | | ln[Odds] |
|---|---|---|---|---|---|---|---|
| | | | DF(Hz) HL | pkR(A.U.) | HL-PSDR (A.U.) | N Shocks before ROSC | |
| Retrospective | VL | Favorable/Favorable | 6.17 | 2 | 6.02 | 1 | -4.88 |
| | L | Favorable/Favorable | 6.02 | 1 | 3.33 | 2 | -2.61 |
| | H | Non-Favorable/Non-Favorable | 2.68 | -1 | 0.67 | 6 | 4.47 |
| | VH | Non-Favorable/Non-Favorable | 2.16 | -1 | 0.31 | 10 | 6.71 |
| Prospective | VL | Favorable/Favorable | 6.59 | 2 | 9.84 | 1 | -6.02 |
| | L | Favorable/Favorable | 4.36 | 1 | 6.54 | 5 | -0.75 |
| | H | Non-Favorable/Non-Favorable | 3.42 | -2 | 0.44 | 1 | 2.9 |
| | VH | Non-Favorable/Non-Favorable | 2.51 | -2 | 0.47 | 11 | 8.02 |

These results clearly illustrate that the present invention can certainly predict neurological performance and/or survival after hospital discharge and/or for at least 6 months after suffering cardiac arrest, in patients which have suffered a cardiac arrest, and optionally comatose status, due to ventricular fibrillation. In particular, the results shown herein demonstrate that the combination of spectral parameters (DF and both HL-PSDR and HLpKR) and optionally the number of shocks delivered before ROSC (NShocks), predict or prognosticate neurological performance and/or survival after hospital discharge and/or for at least 6 months after suffering cardiac arrest, in patients which have suffered a cardiac arrest, and optionally comatose status, due to ventricular fibrillation.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

It will be readily apparent to a person skilled in the art that varying substitutions and modifications may be made to the invention disclosed herein without departing from the scope and spirit of the invention.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should understood that although the present invention has been specifically disclosed by preferred embodiments and optional features, modification and variation of the concepts herein disclosed may be resorted to by those skilled in the art, and that such modifications and variations to be within the scope of this invention as defined by the appended claims.

**Claims**

1. A method of predicting or prognosticating neurological performance in patients which have suffered a cardiac arrest, and optionally comatose status, due to ventricular fibrillation, wherein the method comprises processing at least one electrocardiogram lead from the patient showing the ventricular fibrillation trace prior to the first direct current shock to generate a Power-Spectral-Density (PSD) spectrum, preferably a power spectral density spectrum using the Welch method, and using as an indicator whether the Dominant Frequency value (DF) of the PSD spectrum is at or above 3.9 Hz or below 3.9 Hz in the PSD spectrum.

2. A method of predicting or prognosticating neurological performance and/or survival in patients which have suffered a cardiac arrest, and optionally comatose status, due to ventricular fibrillation, wherein the method comprises processing at least one electrocardiogram lead from the patient showing the ventricular fibrillation trace prior to the first direct current shock, to generate a Power-Spectral-Density (PSD) spectrum, preferably a power spectral density spectrum using the Welch method, and using as indicators: i) whether the Dominant Frequency value (DF) of the PSD spectrum is at or above 3.9 Hz or below 3.9 Hz in the PSD spectrum; ii) the high-to-low power spectral density ratio (HL-PSDR), as the relative power between high (3.9-10 Hz) and low (1.5-3.9 Hz) bands; and iii) the high-to-low peak ratio (HL-pKR), as the relative number of spectral peaks above and below 3.9 Hz with a power above 40% of the DF, from the PSD spectrum.

3. A method of predicting or prognosticating neurological performance and/or survival in patients which have suffered a cardiac arrest, and optionally comatose status, due to ventricular fibrillation, wherein the method comprises processing at least one electrocardiogram lead from the patient showing the ventricular fibrillation trace prior to the first direct current shock, to generate a Power-Spectral-Density (PSD) spectrum, preferably a power spectral density spectrum using the Welch method, and using as indicators: i) whether the Dominant Frequency value (DF) of the PSD spectrum is at or above 3.9 Hz or below 3.9 Hz in the PSD spectrum; ii) the high-to-low power spectral density ratio (HL-PSDR), as the relative power between high (3.9-10 Hz) and low (1.5-3.9 Hz) bands; iii) the high-to-low peak ratio (HL-pKR), as the relative number of spectral peaks above and below 3.9 Hz with a power above 40% of the DF, from the PSD spectrum; and iv) the number of shocks delivered before return of spontaneous circulation (ROSC).

4. A method of predicting or prognosticating neurological performance in patients which have suffered a cardiac arrest, and optionally comatose status, due to ventricular fibrillation, wherein the method comprises:

   a. processing at least one electrocardiogram lead from the patient showing the ventricular fibrillation trace prior to the first direct current shock, to generate a Power-Spectral-Density (PSD) spectrum, preferably a power spectral density spectrum using the Welch method; and
   b. selecting the highest value from the power frequency spectrum as the Dominant Frequency value (DF);

   wherein the result is indicative of a favourable neurological performance if the patient has a Dominant Frequency value (DF) of 3.9 Hz or greater in the PSD spectrum and the result is indicative of a non-favourable neurological performance if the patient has a Dominant Frequency value (DF) of less than 3.9 Hz in the PSD spectrum; wherein favourable neurological performance is understood as cerebral performance categories (CPC) 1 and 2 (good and moderate disability , respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury; and
wherein the DF value is defined as:

$$DF\,(Hz) = \max\{PSD_{II}(f) \in [1.5-10]Hz\}$$

5. A method of predicting or prognosticating neurological performance and/or survival in patients which have suffered a cardiac arrest and comatose status due to ventricular fibrillation, wherein the method comprises:

  a. processing at least one electrocardiogram lead from the patient showing the ventricular fibrillation trace prior to the first direct current shock, to generate a Power-Spectral-Density (PSD) spectrum, preferably a power spectral density spectrum using the Welch method;
  b. selecting the highest value from the power frequency spectrum as the Dominant Frequency value (DF);
  c. selecting the high-to-low power spectral density ratio (HL-PSDR), as the relative power between high (3.9-10 Hz) and low (1.5-3.9 Hz) bands; and
  d. Selecting the high-to-low peak ratio (HL-pKR), as the relative number of spectral peaks above and below 3.9 Hz with a power above 40% of the DF;

  wherein the result is obtained by determining a risk score based on the predictive performance of the combination of at least the three variables above mentioned that classifies the patients in those predicted to have favourable neurological performance or non-favourable neurological performance and/or those predicted to survived or not survived;
  wherein favourable neurological performance is understood as cerebral performance categories (CPC) 1 and 2 (good and moderate disability , respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury;
  wherein survival is understood as the patient being alive for at least 6 months after suffering the aforementioned cardiac arrest;
  wherein the DF value is defined as:

$$DF\,(Hz) = \max\{PSD_{II}(f) \in [1.5-10]Hz\}$$

wherein the high-to-low power spectral density ratio (HL-PSDR) is defined as:

$$HLPSDR(A.U.) = \frac{\left.\int_{Th}^{10Hz} PSD_{II}(f)df\right|_{Th=3.9Hz}}{\left.\int_{1.5}^{Th} PSD_{II}(f)df\right|_{Th=3.9Hz}}$$

and wherein the high-to-low peak ratio (HL-pKR) is defined as:

$$HLPkR(A.U.) = \frac{\left.n\left\{\frac{\partial PSD_{II}(f)}{\partial f}=0, \frac{\partial^2 PSD_{II}(f)}{\partial f^2}<0\right\}\right|_{\substack{PSD(f)>40\%PSD(DF_{max}) \\ f\in[Th,10Hz],Th=3.9Hz}}}{\left.n\left\{\frac{\partial PSD_{II}(f)}{\partial f}=0, \frac{\partial^2 PSD_{II}(f)}{\partial f^2}<0\right\}\right|_{\substack{PSD(f)>40\%PSD(DF_{max}) \\ f\in[1.5Hz,Th],Th=3.9Hz}}}$$

6. A method of predicting or prognosticating neurological performance and/or survival in patients which have suffered a cardiac arrest and comatose status due to ventricular fibrillation, wherein the method comprises:

   a. processing at least one electrocardiogram lead from the patient showing the ventricular fibrillation trace prior to the first direct current shock, to generate a Power-Spectral-Density (PSD) spectrum;
   b. selecting the highest value from the power frequency spectrum as the Dominant Frequency value (DF);
   c. selecting the high-to-low power spectral density ratio (HL-PSDR), as the relative power between high (3.9-10 Hz) and low (1.5-3.9 Hz) bands; and
   d. Selecting the high-to-low peak ratio (HL-pKR), as the relative number of spectral peaks above and below 3.9 Hz with a power above 40% of the DF;

   wherein the result is obtained by using the following formula to determine the risk score (RS):

$$RS = -0.819 \cdot (DF) - 0.023 \cdot (HLPSDR) - 1.236 \cdot (HLPkR) + 4.446$$

   wherein if the risk score is less than 0 the result is indicative of a favourable neurological performance and/or survival and if the risk score value is greater than 0 the result is indicative of a non-favourable neurological performance and non-survival;
   wherein favourable neurological performance is understood as cerebral performance categories (CPC) 1 and 2 (good and moderate disability, respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury;

   wherein survival is understood as the patient being alive for at least 6 months after suffering the aforementioned cardiac arrest;
   wherein the DF value is defined as:

$$DF\,(Hz) = \max\left\{ PSD_{II}(f) \in [1.5 - 10]Hz \right\}$$

   wherein the high-to-low power spectral density ratio (HL-PSDR) is defined as:

$$HLPSDR(A.U.) = \frac{\left. \int_{Th}^{10Hz} PSD_{II}(f)df \right|_{Th=3.9Hz}}{\left. \int_{1.5}^{Th} PSD_{II}(f)df \right|_{Th=3.9Hz}}$$

   wherein the high-to-low peak ratio (HL-pKR) is defined as:

$$HLPkR(A.U.) = \frac{\left. n\left\{ \frac{\partial PSD_{II}(f)}{\partial f} = 0, \frac{\partial^2 PSD_{II}(f)}{\partial f^2} < 0 \right\} \right|_{\substack{PSD(f)>40\%PSD(DF_{max}) \\ f\in[Th,10Hz],Th=3.9Hz}}}{\left. n\left\{ \frac{\partial PSD_{II}(f)}{\partial f} = 0, \frac{\partial^2 PSD_{II}(f)}{\partial f^2} < 0 \right\} \right|_{\substack{PSD(f)>40\%PSD(DF_{max}) \\ f\in[1.5Hz,Th],Th=3.9Hz}}}$$

7. A method of predicting or prognosticating neurological performance and/or survival in patients which have suffered a cardiac arrest, and optionally comatose status, due to ventricular fibrillation, wherein the method comprises:

   a. processing at least one electrocardiogram lead from the patient showing the ventricular fibrillation trace prior to the first direct current shock, to generate a Power-Spectral-Density (PSD) spectrum;
   b. selecting the highest value from the power frequency spectrum as the Dominant Frequency value (DF);

c. selecting the high-to-low power spectral density ratio (HL-PSDR), as the relative power between high (3.9-10 Hz) and low (1.5-3.9 Hz) bands;

d. Selecting the high-to-low peak ratio (HL-pKR), as the relative number of spectral peaks above and below 3.9 Hz with a power above 40% of the DF; and

e. Selecting the number of shocks (NShocks) delivered before return of spontaneous circulation (ROSC);

wherein the result is obtained by determining a risk score based on the predictive performance of the combination of at least the four variables above mentioned that classifies the patients in those predicted to have favourable neurological performance or non-favourable neurological performance and/or those predicted to survived or not survived;

wherein favourable neurological performance is understood as cerebral performance categories (CPC) 1 and 2 (good and moderate disability , respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury;

wherein survival is understood as the patient being alive for at least 6 months after suffering the aforementioned cardiac arrest;

wherein the DF value is defined as:

$$DF(Hz) = \max\left\{ PSD_{II}(f) \in [1.5 - 10]Hz \right\}$$

wherein the high-to-low power spectral density ratio (HL-PSDR) is defined as:

$$HLPSDR(A.U.) = \frac{\left. \int_{Th}^{10Hz} PSD_{II}(f)df \right|_{Th=3.9Hz}}{\left. \int_{1.5}^{Th} PSD_{II}(f)df \right|_{Th=3.9Hz}}$$

and wherein the high-to-low peak ratio (HL-pKR) is defined as:

$$HLPkR(A.U.) = \frac{\left. n\left\{ \frac{\partial PSD_{II}(f)}{\partial f} = 0, \frac{\partial^2 PSD_{II}(f)}{\partial f^2} < 0 \right\} \right|_{\substack{PSD(f)>40\%PSD(DF_{max}) \\ f\in[Th,10Hz],Th=3.9Hz}}}{\left. n\left\{ \frac{\partial PSD_{II}(f)}{\partial f} = 0, \frac{\partial^2 PSD_{II}(f)}{\partial f^2} < 0 \right\} \right|_{\substack{PSD(f)>40\%PSD(DF_{max}) \\ f\in[1.5Hz,Th],Th=3.9Hz}}}$$

8. A method of predicting or prognosticating neurological performance and/or survival in patients which have suffered a cardiac arrest, and optionally comatose status, due to ventricular fibrillation, wherein the method comprises:

a. processing at least one electrocardiogram lead from the patient showing the ventricular fibrillation trace prior to the first direct current shock, to generate a Power-Spectral-Density (PSD) spectrum;

b. selecting the highest value from the power frequency spectrum as the Dominant Frequency value (DF);

c. selecting the high-to-low power spectral density ratio (HL-PSDR), as the relative power between high (3.9-10 Hz) and low (1.5-3.9 Hz) bands;

d. Selecting the high-to-low peak ratio (HL-pKR), as the relative number of spectral peaks above and below 3.9 Hz with a power above 40% of the DF; and

e. Selecting the number of shocks delivered before return of spontaneous circulation (ROSC);

wherein the result is obtained by using the following formula to determine the risk score (RS):

$$RS = -0.734 \cdot (DF) - 0.215 \cdot (HLPSDR) - 1.145 \cdot (HLPkR) + 0.445 \cdot (NShocks) + 2.781$$

wherein if the risk score is less than 0.8 the result is indicative of a favourable neurological performance and/or survival and if the risk score value is greater than 0.8 the result is indicative of a non-favourable neurological performance and non-survival;

wherein favourable neurological performance is understood as cerebral performance categories (CPC) 1 and 2 (good and moderate disability , respectively) according to the patient classification using the Pittsburgh outcome categorization of brain injury, and non-favourable neurological performance is understood as cerebral performance categories (CPC) 3, 4 and 5 (severe disability, vegetative state and brain death, respectively) according to the patient classification using the Pittsburgh outcome categorization of brain injury;

wherein survival is understood as the patient being alive for at least 6 months after suffering the aforementioned cardiac arrest;

wherein the DF value is defined as:

$$DF\,(Hz) = \max\{PSD_{II}(f) \in [1.5-10]Hz\}$$

wherein the high-to-low power spectral density ratio (HL-PSDR) is defined as:

$$HLPSDR(A.U.) = \dfrac{\left.\displaystyle\int_{Th}^{10Hz} PSD_{II}(f)df\right|_{Th=3.9Hz}}{\left.\displaystyle\int_{1.5}^{Th} PSD_{II}(f)df\right|_{Th=3.9Hz}}$$

and wherein the high-to-low peak ratio (HL-pKR) is defined as:

$$HLPkR(A.U.) = \dfrac{\left.n\left\{\dfrac{\partial PSD_{II}(f)}{\partial f}=0, \dfrac{\partial^2 PSD_{II}(f)}{\partial f^2}<0\right\}\right|_{\substack{PSD(f)>40\%PSD(DF_{max})\\f\in[Th,10Hz],Th=3.9Hz}}}{\left.n\left\{\dfrac{\partial PSD_{II}(f)}{\partial f}=0, \dfrac{\partial^2 PSD_{II}(f)}{\partial f^2}<0\right\}\right|_{\substack{PSD(f)>40\%PSD(DF_{max})\\f\in[1.5Hz,Th],Th=3.9Hz}}}$$

9. The method of any of claims 1-8 wherein the patient has suffered a cardiac arrest and comatose status due to ventricular fibrillation.

10. The method of anyone of claims 4 to 9, wherein the dominant frequency in step b) of said method is determined in respect of a period of at least 3 seconds.

11. A computer implemented method, wherein the method is as defined in any of claims 1 to 10.

**Fig. 1**

**Fig. 2**

**Overall Population**
(N=239)
Admitted to ACCU with cardiac arrest and
comatose status undergoing mild hypothermia

**Retrospective**
(n=116)
(Sept 2006 – Sept 2011)

**Prospective**
(n=123)
(Oct 2011 – Jul 2013)

Excluded (n=84)
- 3 Incomplete hypothermia protocol
- 1 Ventricular tachycardia
- 33 Lack of VF tracings (32), tracing artifacts (1)
- 36 Asystole
- 9 Other non-shockable rhythms
- 2 Other (terminal illness, other causes of coma, …)

Excluded (n=94)
- 2 Incomplete hypothermia protocol
- 4 Ventricular tachycardia
- 45 Lack of VF tracings (43), tracing artifacts (2)
- 28 Asystole
- 13 Other non-shockable rhythms
- 2 Other (terminal illness, other causes of coma, …)

Group 1 (n=32)
- 30 Out-of-hospital
- 2 In-hospital

Group 2 (n=29)
- 27 Out-of-hospital
- 2 In-hospital

Fig. 3

A

A1

A2

B

***P<0.001

**Fig. 4**

**Fig. 5**

**Fig. 6**

22.9% (n=14)

8

1.6% (n=1)

1.6% (n=1)

9

0

7

1.6% (n=1)

6

3.3% (n=2)

5 9.8% (n=6)

4 4.9% (n=3)

1

37.7%(n=23)

3

2

6.5% (n=4)

9.8% (n=6)

**1** STEMI
**2** Non STEMI
**3** DCM
**4** HCM
**5** Idiopathic VF
**6** Long QT syndrome
**7** Brugada syndrome
**8** Chronic coronary disease
**9** Wolf-parkinson-white
**0** Severe aortic stenosis

**Fig. 7**

A

FNP+S          FNP+Non-S

R

P

Non-FNP+S      Non-FNP+Non-S

R

P

H   F   H   F

B

3.2 %

Deaths during follow up

6.5 %

Deaths despite favorable
neurological performance

Fig. 8

**Fig. 9**

**Fig. 10**

Fig. 11

Fig. 12

Europäisches
Patentamt

European
Patent Office

Office européen
des brevets

# EUROPEAN SEARCH REPORT

Application Number

EP 14 38 2456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOSHIKAZU GOTO ET AL: "Frequency of ventricular fibrillation as predictor of one-year survival from out-of-hospital cardiac arrests", THE AMERICAN JOURNAL OF CARDIOLOGY, vol. 92, no. 4, 1 August 2003 (2003-08-01), pages 457-459, XP55184111, ISSN: 0002-9149, DOI: 10.1016/S0002-9149(03)00667-2 * page 458, last paragraph - page 459, last paragraph * * page 459, Table 3 *; figure 2 | 1-11 | INV. A61B5/04 A61B5/046 A61B5/00 |
| X | FRED A HAMPRECHT ET AL: "Preliminary results on the prediction of countershock success with fibrillation power", RESUSCITATION, vol. 50, no. 3, 1 September 2001 (2001-09-01), pages 297-299, XP055184212, ISSN: 0300-9572, DOI: 10.1016/S0300-9572(01)00360-4 * abstract * * Table 1 * | 1-11 | |
| X | STROHMENGER H-U ET AL: "Analysis of the Ventricular Fibrillation ECG Signal Amplitude and Frequency Parameters as Predictors of Countershock Success in Humans", CHEST, AMERICAN COLLEGE OF CHEST PHYSICIANS, US, vol. 111, 1 January 1997 (1997-01-01), pages 584-589, XP002317044, ISSN: 0012-3692 * abstract * * page 586, Table 1 * | 1-11 | TECHNICAL FIELDS SEARCHED (IPC)  A61B |

-/--

The present search report has been drawn up for all claims

| Place of search | Date of completion of the search | Examiner |
|---|---|---|
| The Hague | 21 April 2015 | Knüpling, Moritz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 38 2456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STEPHEN LAVER ET AL: "Mode of death after admission to an intensive care unit following cardiac arrest", INTENSIVE CARE MEDICINE, vol. 30, no. 11, 1 November 2004 (2004-11-01), pages 2126-2128, XP055184298, ISSN: 0342-4642, DOI: 10.1007/s00134-004-2425-z * abstract * | 1-11 | |
| A | NOC M ET AL: "ELECTROCARDIOGRAPHIC PREDICTION OF THE SUCCESS OF CARDIAC RESUSCITATION", CRITICAL CARE MEDICINE, LIPPINCOTT WILLIAMS & WILKINS, US, vol. 27, no. 4, 1 April 1999 (1999-04-01), pages 708-714, XP009085286, ISSN: 0090-3493, DOI: 10.1097/00003246-199904000-00021 * page 708, passage bridging column 2 and 3 * * page 709, column 3, lines 25 - 30 * | 1-11 | |
| A | MI HE: "Prediction of Defibrillation Outcome by Ventricular Fibrillation Waveform Analysis: A Clinical Review", JOURNAL OF CLINICAL & EXPERIMENTAL CARDIOLOGY, vol. 01, no. S10, 1 January 2012 (2012-01-01), XP055184337, DOI: 10.4172/2155-9880.S10-009 * abstract * * passage bridging column 1 and 2 of first page * | 1-11 | |

TECHNICAL FIELDS
SEARCHED    (IPC)

The present search report has been drawn up for all claims

| Place of search | Date of completion of the search | Examiner |
|---|---|---|
| The Hague | 21 April 2015 | Knüpling, Moritz |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

3